# EUROPÄISCHE PATENTSCHRIFT

(11) **EP 0 753 509 B1**
(45) Veröffentlichungstag und Bekanntmachung des Hinweises auf die Patenterteilung: **02.11.2000**
(21) Anmeldenummer: 96108860.6
(22) Anmeldetag: 03.06.1996
(51) Int. Cl.: C07C 317/36, C07C 315/06

(54) **Aufarbeitungsverfahren für 4-beta-Sulfatoethylsulfonylanilin-2-sulfonsäure**
Process for the recovery of 4-beta-sulfatoethylsulfonylaniline-2-sulfonic acid
Procédé de récupération d'acide 4-bêta-sulfatoéthylsulfonylaniline-2-sulfonique

(30) Priorität: 14.06.1995 DE 19521620
(43) Veröffentlichungstag der Anmeldung: 15.01.1997
(73) Patentinhaber: DyStar Textilfarben GmbH & Co. Deutschland KG, 60318 Frankfurt am Main (DE)
(72) Erfinder: Jäger, Horst, 51375 Leverkusen (DE)

(56) Entgegenhaltungen:
- DE-A- 2 352 059
- DE-A- 2 538 723
- DE-A- 3 441 273

## Beschreibung

Die Erfindung betrifft ein verbessertes Verfahren zur Aufarbeitung von 4-β-Sulfatoethylsulfonyl-anilin-2-sulfonsäuren.

β-Sulfatoethylsulfonyl-anilin-sulfonsäuren, insbesondere 4-β-Sulfatoethylsulfonyl-anilin-2-sulfonsäuren, sind wichtige Zwischenprodukte, insbesondere zur Herstellung von Reaktivfarbstoffen. Aus der DE-A-2 538 723 (GB-A 1 494 979) ist bereits ein Verfahren zur Herstellung derartiger Verbindungen bekannt, bei dem man eine 1-Aminobenzoldisulfonsäure in 80 bis 96 %iger Schwefelsäure bei Temperaturen von 80 bis 140°C behandelt. Die 1-Aminobenzoldisulfonsäure enthält in 4- oder 5-Stellung einen β-Sulfatoethylsulfonylrest sowie in 2-Stellung und in einer variablen Stellung jeweils eine Sulfonsäuregruppe. Bei dieser Herstellungsweise wird ein Sulfonsäurerest abgespalten. Zur Aufarbeitung der so erhaltenen 1-Aminobenzol-4- und -5-β-Sulfatoethylsulfon-2-sulfonsäuren wird die Sulfierungsschmelze auf Eis ausgetragen und die überschüssige Schwefelsäure mit Alkali- oder Erdalkalihydroxiden neutralisiert und aus dem neutralen Reaktionsgemisch die 4-β-Sulfatoethylsulfon-anilin-2-sulfonsäure durch Aussalzen mit Alkalihalogeniden oder Sprühtrocknen isoliert.In Beispiel 7 wird so gearbeitet, daß die Hauptmenge der überschüssigen Schwefelsäure mit Calciumcarbonat abgestumpft und der Rest mit Soda neutralisiert wird. Man trennt bei 70°C vom ausgefallenen Calciumsulfathalbhydrat durch Filtration ab. Das Natriumsalz der 4-β-Sulfatoethylsulfon-anilin-2-sulfonsäure wird dann durch Eindampfen isoliert.

Diese Aufarbeitung weist Nachteile auf. Aus dem Calciumsulfathalbhydrat (Gips) muß mit reichlich Wasser das Reaktionsprodukt ausgewaschen werden. Dadurch fällt es in einer sehr verdünnten Lösung an, aus der es nur durch Zufuhr thermischer Energie (Eindampfen) als Feststoff isoliert werden kann. Weiterhin entstehen große Mengen an Gips, die deponiert werden müssen.

Der Erfindung liegt die Aufgabe zugrunde, ein verbessertes Aufarbeitungsverfahren zur Verfügung zu stellen.

Die Erfindung betrifft ein verbessertes Verfahren zur Herstellung der Verbindung der allgemeinen Formel (1a) in Form des Dikaliumsalzes, wobei die Verbindung (Ia) als Lösung in wäßriger Schwefelsäure vorliegt, 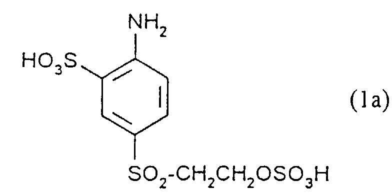 dadurch gekennzeichnet, daß man die Verbindung der allgemeinen Formel (1a) mit Kalisalzen abscheidet. Dabei fällt das Reaktionsprodukt in einer sehr guten Saugform an, wobei gleichzeitig eine Reinigung eintritt, da Spuren von Nebenprodukten mit der Mutterlauge entfernt werden. Als Kaliumsalze kommen Kaliumacetat, Kaliumphosphate, Kaliumsulfat und insbesondere Kaliumchlorid infrage.

Die wäßrige Schwefelsäure weist eine Säurekonzentration von 5 bis 80 %, vorzugsweise von 5 bis 70% insbesondere 20 bis 50 % auf.

Pro Mol Ausgangsmaterial werden 1 bis 10 Mol Moläquivalente Kaliumsalz zugesalzt. Bevorzugt ist die Verwendung von 2 bis 3 Moläquivalenten.

Bei der Herstellung der Verbindung (1a) fällt bei Zugabe von Kaliumsalz zu der wäßrigen Schwefelsäure die Verbindung als Dikaliumsalz aus.

Aus DE-A 23 52 059 ist 1-Amino-5-beta-sulfatoethylsulfon-2,4-disulfonsäure, deren 5-Vinylsulfonverbindung und deren Alkalisalze sowie Verfahren zu ihrer Herstellung bekannt.

DE-A 34 41 273 beschreibt Monocyclische Bis-oxethylsulfonyl-aniline sowie Verfahren zu deren Herstellung.

### Beispiel

a) Sulfierung
   In eine Vorlage aus 230 ml = 437 g 20 %igem Oleum werden bei Raumtemperatur beginnend 142 g = 0,485 Mol 96 %iges 4-β-Sulfatoethylsulfonyl-anilin eingetragen, wobei die Temperatur auf 48°C ansteigt. Anschließend erwärmt man die Lösung drei Stunden auf 115 ± 2°C.
b) Desulfonierung
   Die Sulfierung wird auf 70 bis 80°C abgekühlt. Man tropft dann 42 g 50 gew.-%ige Schwefelsäure ein, wobei die Temperatur nicht über 100°C ansteigen soll. Anschließend erwärmt man zehn Stunden auf 95 bis 100°C.
c) Isolierung
   Die Schmelze wird auf 40 bis 50°C abgekühlt und unter Kühlung von außen auf ein Gemisch von 700 g Eiswasser gegeben. Dabei soll die Temperatur nicht über 20°C ansteigen. Zu der resultierenden klaren Lösung werden 75 g = 1,0 Mol Kaliumchlorid gegeben. Man rührt drei Stunden nach und saugt das ausgeschiedene Reaktionsprodukt ab.
   Der Rückstand wiegt 282 g und ist 57,5 %ig, bezogen auf Mol 361 4-β-Sulfatoethylsulfonyl-anilin-2-sulfonsäure.

Das Produkt kann durch Umkristallisation aus Wasser (bei 70°C lösen und bei 0 bis 5°C absaugen) gereinigt werden.

| **Elementaranalyse** C₈H₁₁NO₁₀S₃K₂ | | | | | | |
|---|---|---|---|---|---|---|
| | C | H | N | O | S | K |
| ber. | 21,1 % | 2,4 % | 3,1 % | 35,2 % | 21,1 % | 17,1 % |
| gef. | 21,1 | 2,4 | 3,0 | - | 21,4 | 16,9 |

## Patentansprüche

1. Verfahren zur Isolierung der Verbindung der Formel (Ia) in Form ihres Dikaliumsalzes 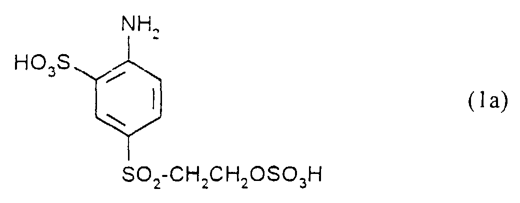 wobei die Verbindung (Ia) als Lösung in wäßriger Schwefelsäure vorliegt, dadurch gekennzeichnet, daß die wäßrige Schwefelsäure eine Säurekonzentration von 5 bis 80 % aufweist und daß man die Verbindung (Ia) durch Zusatz von Kaliumsalzen abscheidet, wobei man pro Mol der Verbindung (Ia) 1 bis 10, Moläquivalente Kaliumsalz verwendet.

2. Verfahren nach Anspruch 1, dadurch gekennzeichnet, daß man als Kaliumsalz Kaliumacetat, Kaliumphosphat, Kaliumsulfat oder Kaliumchlorid verwendet.

3. Verfahren nach wenigstens einem der vorhergehenden Ansprüche, dadurch gekennzeichnet, daß die wäßrige Schwefelsäure eine Säurekonzentration von 20 bis 50 %, aufweist.

4. Verfahren nach wenigstens einem der vorhergehenden Ansprüche, dadurch gekennzeichnet, daß man pro Mol der Verbindung (Ia) 2 bis 3 Moläquivalente Kaliumsalz verwendet.

## Claims

1. Process for the isolation of the compound of the formula (Ia) in the form of its dipotassium salt 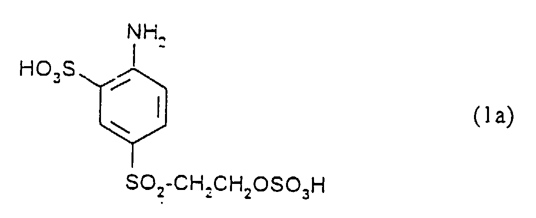 the compound (Ia) being present as a solution in aqueous sulphuric acid, characterized in that the aqueous sulphuric acid has an acid concentration of 5 to 80% and in that the salt of the compound (Ia) is deposited by addition of potassium salts, 1 to 10 molar equivalents of potassium salt being used per mole of the compound (Ia).

2. Process according to Claim 1, characterized in that the potassium salt used is potassium acetate, potassium phosphate, potassium sulphate or potassium chloride.

3. Process according to at least one of the preceding claims, characterized in that the aqueous sulphuric acid has an acid concentration of 20 to 50%.

4. Process according to at least one of the preceding claims, characterized in that 2 to 3 molar equivalents of potassium salt are used per mole of the compound (Ia).

## Revendications

1. Procédé pour isoler le composé de formule (Ia) sous forme de son sel dipotassique 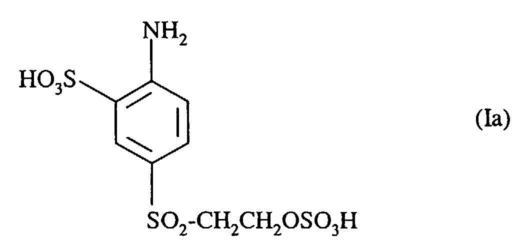 où le composé (Ia) est sous forme de solution dans l'acide sulfurique aqueux, caractérisé en ce que l'acide sulfurique aqueux présente une centration d'acide de 5 à 80 % et en ce que l'on sépare le composé (Ia) par addition de sels de potassium, en utilisant par mole de composé (Ia) 1 à 10 équivalents molaires de sel de potassium.

2. Procédé selon la revendication 1, caractérisé en ce que l'on utilise comme sel de potassium l'acétate de potassium, le phosphate de potassium, le sulfate de potassium ou le chlorure de potassium.

3. Procédé selon au moins l'une des revendications précédentes, caractérisé en ce que l'acide sulfurique aqueux présente une concentration d'acide de 20 à 50 %.

4. Procédé selon au moins l'une des revendications précédentes, caractérisé en ce que l'on utilise par mole de composé (la) 2 à 3 équivalents molaires de sel de potassium.
